# EUROPEAN PATENT APPLICATION

(11) **EP 4 401 085 A1**
(43) Date of publication of application: **17.07.2024**
(21) Application number: 24151616.0
(22) Date of filing: 12.01.2024
(51) Int. Cl.: G16H 20/17, A61M 5/168, G16H 40/63

(54) **LIQUID INFUSION CONTROL METHOD AND APPARATUS, ELECTRONIC DEVICE AND STORAGE MEDIUM**

(30) Priority: 12.01.2023 CN 202310072905
(71) Applicant: Medcaptain Medical Technology Co., Ltd., Shenzhen, Guangdong 518000 (CN)
(72) Inventor: Li, Ping, Guangdong, 518000 (CN); Wu, Longyu, Guangdong, 518000 (CN); Tang, Yiling, Guangdong, 518000 (CN); Tang, Yazhou, Guangdong, 518000 (CN)
(74) Representative: Grünecker Patent- und Rechtsanwälte PartG mbB

(57) **Abstract**

The control method applied to a liquid infusion system includes: receiving a command delivered by the input device and obtaining an infusion attribute information from the input device depending on the command; the infusion attribute information includes parameter information, and the parameter information comprises a total intake for a predetermined time period and at least one first intake corresponding to the at least one first liquid; obtaining at least one second intake corresponding to the at least one second liquid based on the total intake and the at least one first intake; controlling at least one infusion pump with the first liquid to infuse the first liquid into the target based on the at least one first intake ; and controlling at least one infusion pump with the second liquid to infuse the second liquid to the target based on the at least one second intake.

## Description

### TECHNICAL FIELD

The present disclosure relates to the field of a medical device, and more particularly, to a liquid infusion control method and apparatus, electronic device and storage medium.

### BACKGROUND

Different targets will have different daily liquid intakes, and a critical care unit will strictly control the total liquid intake of the target, sometimes less (such as critically ill patients with metabolic problems) and sometimes more (such as patients prior to undergoing organ transplantation), but no matter what the target is, there will be a 24-hour liquid intake index, so that the medical staff member can manage the liquid balance of the patient.

However, the inventor realizes that all parameters of the infusion liquid in current liquid infusion systems are entered manually by the medical staff in sequence, and mainly rely on the medical staff to manually calculate and input the intake of each liquid in the liquid infusion system according to the results of the calculations. It is not only prone to low accuracy due to calculation errors made by the medical staff, but also prone to the problem of inefficiency and untimely execution due to the tedious setting of the parameters carried out by manual personnel.

### SUMMARY

The embodiments of the invention provide a liquid infusion control method and apparatus, electronic device and storage medium to solve a problem of poor accuracy of liquid intakes due to manual calculation and input, which is easily caused by miscalculations by a medical staff member, and inefficiency and untimely execution due to the tedious setting of the parameters carried out by the medical staff member.

In a first aspect, the invention provides a liquid infusion control method applied to a liquid delivery system, in which the liquid infusion system comprises: a control device, an input device and an infusion pump, the method runs in the control device and comprises:
receiving a command delivered by the input device and obtaining an infusion attribute information from the input device according to the command; wherein the command indicates that the input device has generated an infusion attribute information, and the infusion attribute information indicates an infusion of at least one first liquid and at least one second liquid into the target; each first liquid is kept in one infusion pump, and each second liquid is kept in another infusion pump; the infusion attribute information comprises parameter information, and the parameter information comprises a total intake for a predetermined time period and at least one first intake corresponding to the at least one first liquid; the first intake defines an infusion volume of the first liquid infused into the target, and the total intake defines an infusion volume of the at least one first liquid and the at least one second liquid infused into the target;
obtaining at least one second intake corresponding to the at least one second liquid based on the total intake and the at least one first intake; wherein the second intake defines an infusion volume of the second liquid infused into the target;
controlling at least one infusion pump with the first liquid to infuse the first liquid into the target based on the at least one first intake ; and
controlling at least one infusion pump with the second liquid to infuse the second liquid to the target based on the at least one second intake.

In the above embodiment, the step of obtaining at least one second intake corresponding to the at least one second liquid based on the total intake and the at least one first intake, further comprises:
calculating the sum of the at least one first intake to obtain a total amount of the first liquid;
subtracting the total intake from the total amount of the first liquid to obtain a total amount of the second liquid;
obtaining a proportional information from the infusion attribute information and determining a second intake of each second liquid respectively; wherein the proportion information defines a proportion of each second liquid infused into the target.

In the above embodiment, the step of controlling at least one infusion pump with the first liquid to infuse the first liquid to the target based on the at least one first intake comprises:
obtaining a first infusion time of each first liquid respectively based on a first flow rate and the first intake corresponding to each first liquid when the parameter information comprises the first flow rate;
controlling the infusion pump with the first liquid to infuse the first liquid into the target based on the first infusion time and the first flow rate; and
the step of controlling at least one infusion pump with the second liquid to infuse the second liquid to the target based on the at least one second intake, comprises:
   obtaining a second infusion time of each second liquid respectively based on a second flow rate and the second intake corresponding to each second liquid when the parameter information comprises the second flow rate;
   controlling the infusion pump with the second liquid to infuse the second liquid into the target based on the second infusion time and the second flow rate;
   wherein the first flow rate is a speed of the first liquid infused into the target, and the second flow rate is a speed of the second liquid infused into the target.

In the above embodiment, the step of controlling at least one infusion pump with the first liquid to infuse the first liquid to the target based on the at least one first intake comprises:
obtaining a first flow rate of each first liquid respectively based on a duration of a first infusion time period and the first intake corresponding to each first liquid when the parameter information comprises the first infusion time period;
controlling the infusion pump with the first liquid to infuse the first liquid into the target based on the first infusion time period and the first flow rate; and
the step of controlling at least one infusion pump with the second liquid to infuse the second liquid to the target based on the at least one second intake, comprises:
   obtaining a second flow rate of each second liquid respectively based on a duration of a second infusion time period and the second intake corresponding to each second liquid when the parameter information comprises the second infusion time period;
   controlling the infusion pump with the second liquid to infuse the second liquid into the target based on the second infusion time period and the second flow rate.

In the above embodiment, the liquid infusion system comprises a timer corresponding to each infusion pump;
after the step of controlling at least one infusion pump with the first liquid to infuse the first liquid to the target based on the at least one first intake; and controlling at least one infusion pump with the second liquid to infuse the second liquid to the target based on the at least one second intake, the method further comprises:
obtaining a first duration of each first liquid respectively from the timer of the infusion pump with the first liquid; and obtaining a first infused volume of each first liquid respectively based on the first duration and the first flow rate corresponding to each first liquid; wherein the first duration defines an infused time of the first liquid infused into the target, and the first infused volume defines a liquid volume of the first liquid infused into the target; and
obtaining a second duration of each second liquid respectively from the timer of the infusion pump with the second liquid; and obtaining a second infused volume of each second liquid respectively based on the second duration and the second flow rate corresponding to each second liquid; wherein the second duration defines an infused time of the second liquid infused into the target, and the second infused volume defines a liquid volume of the second liquid infused into the target.

In the above embodiment, the liquid infusion system comprises a timer corresponding to each infusion pump;
after the step of controlling at least one infusion pump with the first liquid to infuse the first liquid to the target; and controlling at least one infusion pump with the second liquid to infuse the second liquid to the target, the method further comprises:
receiving at least one first update name and its first update intake sent by the input device;
setting the first liquid corresponding to the first update name as a first target liquid, and controlling the infusion pump with the first target liquid to infuse the first target liquid into the target based on the first update intake corresponding to the first update name of the first liquid ;
obtaining a first update difference based on the first intake and the first update intake of the at least one first target liquid, and obtaining a second update intake of the at least one second liquid by adjusting the second intake of the at least one second liquid according to the first update difference, and controlling the infusion pump with the second liquid to infuse the second liquid into the target based on the second update intake.

In the above embodiment, the step of controlling the infusion pump with the first target liquid to infuse the first target liquid into the target based on the first update intake corresponding to the first update name of the first liquid comprises:
obtaining a first duration of each first target liquid respectively from the timer corresponding to each infusion pump with the first target liquid, and obtaining a first infused volume of each first target liquid respectively based on the first duration and the first flow rate of each first target liquid; wherein the first duration defines an infused time of the first target liquid infused into the target, and the first infused volume defines a liquid volume of the first target liquid infused into the target;
obtaining a first volume to be infused of each first target liquid respectively based on the first update intake and the first infused volume of each first target liquid; wherein the first volume to be infused defines a remaining volume of the first target liquid infused into the target;
obtaining a first update time period of each first target liquid respectively based on the first flow rate and the first volume to be infused; and controlling the infusion pump with the first target liquid to infuse each first target liquid with the remaining volume into the target during the first update time period according to the first flow rate; or
calculating a first time difference between the duration of the first infusion time period and the first duration, and obtaining a first update flow rate of the first target liquid based on the first volume to be infused and the first time difference, and controlling the infusion pump with the first target liquid infuse each first target liquid with the remaining volume into the target during the first time difference according to the first update flow rate.

In the above embodiment, the step of obtaining the first update difference based on the first intake and the first update intake of the at least one first target liquid, further comprises:
calculating a sum of each first intake of the at least one first liquid to obtain an original total intake of the at least one first liquid;
calculating a sum of each non-updated first intake and each first updated intake of the at least one first liquid or calculating the sum of each first updated intake to obtain an updated total intake of the at least one first liquid;
calculating a difference between the original total intake and the updated total intake to obtain the first update difference.

In the above embodiment, the step of obtaining the second update intake of the at least one second liquid by adjusting the second intake of the at least one second liquid according to the first update difference comprises:
setting a corresponding second liquid as a second target liquid according to a preset dynamic rule or a received adjustment request;
obtaining the second update intake of each second target liquid respectively by adjusting the intake of the second target liquid according to the first update difference.

In the above embodiment, the step of controlling the infusion pump with the second liquid to infuse the second liquid into the target based on the second update intake, further comprises:
obtaining a second duration of each second liquid respectively from the timer corresponding to each infusion pump with the second liquid, and obtaining a second infused volume of each second liquid respectively based on the second duration and the second flow rate of each second liquid; wherein the second duration defines an infused time of the second liquid infused into the target, and the second infused volume defines a liquid volume of the second liquid infused into the target;
obtaining a second volume to be infused of each second liquid respectively based on the second update intake and the second infused volume of each second liquid; wherein the second volume to be infused defines a remaining volume of the second liquid infused to the target;
obtaining a second update time period of each second liquid respectively based on the second flow rate and the second volume to be infused; and controlling the infusion pump with the second liquid to infuse each second liquid with the remaining volume into the target during the second update time period according to the second flow rate; or
calculating a second time difference between the length of the second infusion time period and the second duration, and obtaining a second update flow rate of the second liquid based on the second volume to be infused and the second time difference, and controlling the infusion pump with the second liquid infuse each second liquid with the remaining volume into the target during the second time difference according to the second update flow rate.

In a second aspect, the invention provides a liquid infusion control apparatus applied to a liquid infusion system, the liquid infusion system comprises a control device, an input device and an infusion pump; and the apparatus is installed in the control device and comprises:
an input module configured to receive a command delivered by the input device and obtain an infusion attribute information from the input device depending on the command; wherein the command indicates that the input device has generated an infusion attribute information, and the infusion attribute information is configured to indicate an infusion of at least one first liquid and at least one second liquid into the target; each first liquid is kept in one infusion pump, and each second liquid is kept in another infusion pump; the infusion attribute information comprises parameter information, and the parameter information comprises a total intake for a predetermined time period and at least one first intake corresponding to the at least one first liquid; the first intake defines an infusion volume of the first liquid infused into the target, and the total intake defines an infusion volume of the at least one first liquid and the at least one second liquid infused into the target;
a processing module configured to obtain at least one second intake corresponding to the at least one second liquid based on the total intake and the at least one first intake; wherein the second intake defines an infusion volume of the second liquid infused into the target;
an execution module configured to control at least one infusion pump with the first liquid to infuse the first liquid to the target based on the at least one first intake and control at least one infusion pump with the second liquid to infuse the second liquid to the target based on the at least one second intake.

In a third aspect, the invention provides an electronic device, comprising a processor and a memory communicated with the processor; wherein
the memory stores computer-executable instructions; and
the processor is configured to execute the computer-executable instructions to implement the method as described in the first aspect of the invention.

In a fourth aspect, the invention provides a computer-readable storage medium, wherein the computer-readable storage medium stores a computer program, the computer program being executed by a processor to implement the method as described in the first aspect.

In a fifth aspect, the invention provides a computer program product comprising a computer program, wherein the computer program when executed by a processor implements the liquid infusion control method described above.

The invention provides a liquid infusion control method and apparatus, electronic device and storage medium, in which at least one second intake corresponding to the at least one second liquid may be determined based on the total intake and the at least one first intake in the infusion attribute information. The second intake of the second liquid can be automatically generated without the need for the medical staff member to manually adjust the second intake of each second liquid, thereby improving the efficiency and accuracy of the liquid intake setting operation.

The at least one first liquid and the at least one second liquid are respectively infused into the target according to the at least one first intake and the at least one second intake, thereby realizing the technical effect of automatically controlling the infusion pump to carry out the infusion, and avoiding the occurrence of the problem of manually carrying out the parameter setting as well as the operation of the execution, which leads to the problem of low efficiency of the operation and the problem of not being carried out in a timely manner.

### BRIEF DESCRIPTION OF THE DRAWINGS

The accompanying drawings herein, which are incorporated into and form a part of the specification, illustrate embodiments consistent with the present disclosure and are used in conjunction with the specification to explain the principles of the present application.
FIG 1 depicts an application scenario provided in an embodiment of the invention.
FIG 2 shows a flowchart of a liquid infusion control method in Embodiment 1 of the invention.
FIG 3 shows a flowchart of a liquid infusion control method in Embodiment 2 of the invention.
FIG 4 shows a flowchart of a liquid infusion control method in Embodiment 3 of the invention.
FIG 5 shows a program module of a liquid infusion control apparatus of the invention.
FIG 6 depicts a hardware structure of an electronic device in an electronic device of the invention.

Embodiments of the invention shown in the above drawings, which will be described in more detail later. These accompanying drawings and textual descriptions are not intended to limit the scope of the concept of the invention in any way, but rather to illustrate the concepts of the invention for those skilled in the art by reference to particular embodiments.

### DETAILED DESCRIPTION

Examples of embodiments will be described herein in detail, examples of which are represented in the accompanying drawings. When the following description relates to the accompanying drawings, the same numerals in the different accompanying drawings indicate the same or similar elements unless otherwise indicated. The embodiments described in the following examples do not represent all embodiments consistent with the invention. Rather, they are only examples of apparatuses and methods consistent with some aspects of this invention as detailed in the appended claims.

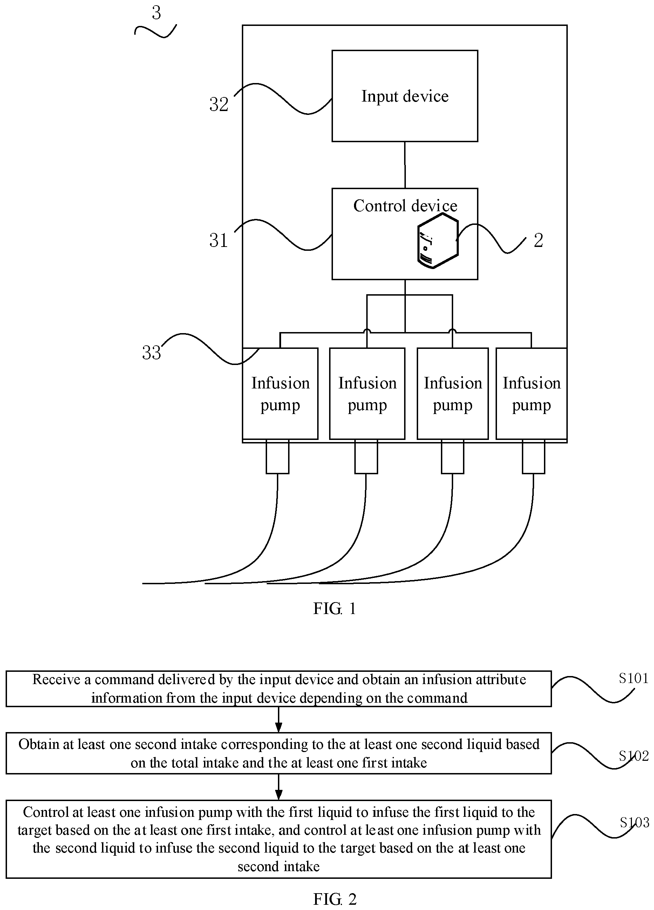

As shown in FIG 1, a specific application scenario of the invention may be a control module 2 running a liquid infusion control method, and the control module 2 may be installed in a liquid infusion system 3. The liquid infusion system 3 includes a control device 31, an input device 32 and an infusion pump 33. The control module 2 receives a command delivered by the input device 32 and obtains infusion attribute information from the input device depending on the command. Based on a total intake and at least one first intake, the control module 2 may obtain at least one second intake corresponding to at least one second liquid. The second intake defines an infusion volume of the second liquid infused into the target. The control module 2 controls at least one infusion pump 33 provided with the first liquid to infuse at least one first liquid to the target by means of the control device 31. And the control module 2 controls at least one infusion pump 33 provided with the second liquid to infuse at least one second liquid to the target by means of the control device 31.

The target may be a patient for receiving an infusion, an animal for receiving an infusion, and a dispensing system for receiving a first liquid and a second liquid from the liquid infusion system.

Further, the control module 2 running a liquid infusion control method may be arranged in an infusion workstation or a central station having at least one liquid infusion system 3. The control module 2 is configured to carry out unified management of the at least one liquid infusion system 3 and control each liquid infusion system 3 to infuse the at least one first liquid and the at least one second liquid into the target corresponding thereto.

The technical solutions of the invention and how the technical solutions of the invention solve prior art problems are described in detail below with reference to specific embodiments. The following specific embodiments may be combined with each other, and the same or similar concepts or processes may not be repeated in the description of certain embodiments. Embodiments of the invention will be described below in conjunction with the accompanying drawings.

### Embodiment 1

As shown in FIG 2, the invention provides a liquid infusion control method applied to a liquid infusion system. The liquid infusion system includes a control device, an input device and an infusion pump. The method runs in the control device and includes the following steps:
S101: When a command delivered by the input device is received, infusion attribute information may be obtained from the input device depending on the command. The command indicates that the input device has generated infusion attribute information. The infusion attribute information indicates an infusion of at least one first liquid and at least one second liquid into the target. Each first liquid is kept in one infusion pump, and each second liquid is kept in another infusion pump. The infusion attribute information includes parameter information, and the parameter information includes a total intake for a predetermined time period and at least one first intake corresponding to the at least one first liquid. The first intake defines an infusion volume of the first liquid infused into the target. The total intake defines an infusion volume of the at least one first liquid and the at least one second liquid infused into the target.

In this step, the input device is a device for a medical staff member to input parameter information and is one of the main devices for exchanging information between the medical staff member and the computer system. The input device includes, but is not limited to, a keyboard, a mouse, a touchscreen, a scanner, a light pen, a stylus input board, a joystick or a voice input device. In this embodiment, an input device is a device by which a person or an external party interacts with a computer, and the input device is configured to enter raw data and commands to process the data into a computer. The computer is capable of receiving a wide variety of data, both numerical and non-numerical, and the data, such as graphics, images, sound, may be input into the computer through different types of input devices for storage, processing and output.

A control device is a control module or control circuit of a liquid infusion system to control the flow rate and infusion time of an infusion pump based on an input device.

An infusion pump is an intelligent infusion device, and the infusion pump is configured to hold one first liquid or one second liquid. The infusion pump is a specialized medical device that uses a mechanical drive to accurately control the number of drops or the flow rate of the infusion to ensure that the dose enters the patient's body accurately and safely. The use of infusion pumps in the clinic has greatly improved the accuracy, safety and quality of care.

Optionally, before the step of obtaining the infusion attribute information, the method may further includes the following steps:
At least one item of ingredient information sent by the input device is received. The ingredient information is the name of the first liquid and/or the second liquid.

At least one first intake and the total intake within a predetermined time period sent by the input device are received. The parameter information may be determined by aggregating the total intake and the at least one first intake. One first intake corresponds to one first liquid.

The infusion attribute information may be determined based on the ingredient information and the parameter information.

The command sent by the input device may be received based on the infusion attribute information.

Accordingly, the medical staff member enters, by means of the input device, the ingredient information of each first liquid and each second liquid, as well as the total intake of all first liquids and all second liquids to be infused into the target. At the same time, the medical staff member may also enter the intake corresponding to each first liquid to be infused into the target, so that the medical staff member can control the intake of the first liquid.

Optionally, the step of receiving the at least one ingredient information sent by the input device may include the following steps:
Receiving ingredient information sent by the input device;
Comparing the ingredient information to a preset first liquid table and a preset second liquid table, respectively, in which the first liquid table has at least one name of the first liquid and the second liquid table has at least one name of the second liquid;
Determining the ingredient information to be the name of the first liquid when the ingredient information belongs to the first liquid table;
Determining the ingredient information to be the name of the second liquid when the ingredient information belongs to the second liquid table.

In this embodiment, the first liquid is a primary drug (master drug) infused into the patient by the infusion pump of the liquid infusion system; the first liquid may be a vasoactive drug, insulin, sedative drug, and the like. The second liquid is a secondary drug (slave drug) infused into the patient by the infusion pump of the liquid infusion system; the second liquid may be saline, glucose, and the like.

Specifically, the step of determining the infusion attribute information based on the ingredient information and the parameter information includes the following steps:
Receiving at least one first flow rate and at least one second flow rate sent by the input device are, loading the at least one first flow rate and the at least one second flow rate as parameter information, and determining the infusion attribute information based on the ingredient information and the parameter information.

Instead, or in addition, when at least one first infusion period and at least one second infusion period sent by the input device are received, the at least one first infusion period and the at least one second infusion period will be loaded as the parameter information, and the infusion attribute information will be determined based on the ingredient information and the parameter information.

Specifically, the step of determining the infusion attribute information based on the ingredient information and the parameter information is further followed by the following step:
Receiving the execution time sent by the input device; and
Loading both the infusion attribute information and the execution time into a preset infusion list, such that the infusion attribute information can be correlated with the execution time.

Preferably, the step of obtaining the infusion attribute information may include the following steps:
Obtaining an infusion list, in which the infusion list includes at least one item of infusion attribute information and an execution time corresponding to the infusion attribute information, and the execution time defines a time to infuse the first liquid into the target and a time to infuse the second liquid into the target;
Polling the execution time in the infusion list, and if it is confirmed that there is an execution time in the infusion list that corresponds to the current time, setting the execution time that corresponds to the current time as the target time, and obtaining the infusion attribute information corresponding to the target time.

In this embodiment, the liquid infusion system has different execution times for the infusion of the first liquid and the second liquid. If the current time reaches the execution time of a particular first liquid or second liquid, the first liquid or second liquid will be infused into the patient, ensuring controllable infusion of the first liquid and the second liquid.

S102: at least one second intake corresponding to the at least one second liquid may be obtained based on the total intake and the at least one first intake. The second intake defines an infusion volume of the second liquid infused into the target.

In this step, the second intake of each second liquid may be determined respectively based on the total intake and the at least one first intake. As a result, the second intake can be generated automatically without the need for manual adjustment by a medical staff member, thereby improving the efficiency and accuracy of liquid intake setting operations.

In one embodiment, the step of obtaining at least one second intake based on the total intake and the at least one first intake comprises the following steps:
Obtaining the total amount of the first liquids by calculating the sum of the at least one first intake;
Obtaining the total amount of the second liquids by subtracting the total intake from the total amount of the first liquids;
Obtaining proportional information from the infusion attribute information. The intake of each second liquid may be determined respectively based on the proportional information and the total amount of the second liquids. The proportional information defines the proportion of each second liquid infused to the target.

In this embodiment, the sum of the at least one first intake is calculated to obtain the total amount of the first liquid, and then the total amount of the second liquid is determined by subtracting the total intake from the total amount of the first liquid.

The intake of each second liquid may be determined respectively based on the total amount of the second liquid described above and the preset proportional information.

If the proportional information is 50% glucose and 50% saline, and the total amount of the second liquid is 1L, then it can be determined that the second liquid includes glucose and saline, and the second intake of glucose is 500 ml and the second intake of saline is 500 ml.

If the proportional information is 100% glucose, and the total amount of the second liquid is 1L, then it can be determined that the second liquid only includes glucose, and the second intake of glucose is 1 L.

In this embodiment, the proportional information may be set by the medical staff member as needed.

Therefore, assigning the intake of each second liquid respectively based on the proportional information set by the medical staff member and total amount of the second liquids improves the efficiency and accuracy of the calculation of the intake of each second liquid.

S 103 : at least one infusion pump with the first liquid is controlled to infuse the first liquid into the target based on the at least one first intake, and at least one infusion pump with the second liquid infuses the second liquid into the target based on the at least one second intake.

In this step, the liquid infusion system infuses the at least one first liquid and the at least one second liquid into the target according to the respective intakes, thereby realizing automatic control of the infusion pump of the liquid infusion system to carry out the infusion, and avoiding the occurrence of the problem of manually carrying out the cumbersome parameter setting that leads to the inefficiency of the operation and the lack of timely execution.

In one embodiment, the step of controlling at least one infusion pump with the first liquid to infuse the first liquid into the target based on the at least one first intake may include the following steps:
When the parameter information includes a first flow rate corresponding to each first liquid, obtaining a first infusion time corresponding to each first liquid respectively based on the first flow rate and the first intake of the first liquid, and controlling the infusion pump with the first liquid to infuse the first liquid into the target based on the first infusion time and the first flow rate.

The step of controlling at least one infusion pump with the second liquid to infuse the second liquid to the target based on the at least one second intake may include the following steps:
When the parameter information includes a second flow rate corresponding to each second liquid, obtaining a second infusion time corresponding to each second liquid respectively based on the second flow rate and the second intake of the second liquid, and controlling the infusion pump with the second liquid to infuse the second liquid into the target based on the second infusion time and the second flow rate.

The first flow rate is a speed at which the first liquid is infused into the target. The second flow rate is a speed at which the second liquid is infused into the target.

In this embodiment, if a first flow rate corresponding to each first liquid is defined in the parameter information, the first infusion time of the first liquid may be determined based on the first intake and the first flow rate of the first liquid. For example, if the first intake is 500 ml and the first flow rate is 5 ml/min, the first infusion time is determined to be 100 min. The infusion pump of the liquid infusion system will be controlled by the first flow rate and the first infusion time so that the infusion pump may infuse the first liquid into the patient at a flow rate of 5 ml/min within 100 min.

If a second flow rate corresponding to each second liquid is defined in the parameter information, the second infusion time of the second liquid may be determined based on the second intake and the second flow rate of the second liquid. For example, if the second intake is 1 L and the second flow rate is 10 ml/min, the second infusion time is determined to be 100 min. The infusion pump of the liquid infusion system may then be controlled by the second flow rate and the second infusion time so that the infusion pump may infuse the second liquid into the patient at a flow rate of 10 ml/min within 100 min.

In one embodiment, the step of controlling at least one infusion pump with the first liquid to infuse the first liquid into the target based on the at least one first intake may include the following steps:
When the parameter information includes a first infusion time period corresponding to each first liquid, obtaining a first flow rate corresponding to each first liquid respectively based on the duration of the first infusion time period and the first intake of the first liquid, and controlling the infusion pump with the first liquid to infuse the first liquid into the target based on the first infusion time period and the first flow rate.

The step of controlling at least one infusion pump with the second liquid to infuse the second liquid to the target based on the at least one second intake may include the following steps:
When the parameter information includes a second infusion time period corresponding to each second liquid, obtaining a second flow rate corresponding to each second liquid respectively based on the duration of the second infusion time period and the second intake of the second liquid, and controlling the infusion pump with the second liquid may infuse the second liquid into the target based on the second infusion time period and the second flow rate.

In this embodiment, if a first infusion time period corresponding to each first liquid is defined in the parameter information, the first flow rate of the first liquid may be determined based on the first intake and the first infusion time period of the first liquid. For example, if the first infusion time period is 9:00-10:00, the duration of the first infusion period may be 60 min, and if the first intake is 60 ml, the first flow rate may be 1 ml/min. Then the liquid infusion system may deliver the first liquid to the patient at a flow rate of 1 ml/min via the infusion pump during the 9:00-10:00 time period.

In this embodiment, if a second infusion time period corresponding to each second liquid is defined in the parameter information, the second flow rate of the second liquid may be determined based on the second intake and the second infusion time period of the second liquid. For example, if the second infusion time period is 9:00-11:00, the duration of the second infusion period may be 120 min, and if the second intake is 180 ml, the second flow rate may be 1.5 ml/min. Then the liquid infusion system may deliver the second liquid to the patient at a flow rate of 1.5 ml/min via the infusion pump during the 9:00-11:00 time period.

Further, the step of obtaining the first flow rate corresponding to each first liquid respectively based on the duration of the first infusion time period and the first intake of the first liquid may include the following steps:
Obtaining the duration of the first infusion period by calculating the sum of the at least one first time period, in which one first time period defines an uninterrupted interval of time for infusing the first liquid into the target; and
Obtaining the first flow rate of each first liquid by dividing the first intake and the duration of the first infusion period of the first liquid.

In this embodiment, when the first infusion time period includes two first time periods, and the two first time periods are 9:00-10:00 and 14:00-15:00, the duration of the first infusion period may be 120 min. If the first intake is 60 ml, the first flow rate may be 0.5 ml/min. Then the liquid infusion system may deliver the first liquid to the patient at a flow rate of 0.5 ml/min via the infusion pump during two time periods, 9:00-10:00 and 14:00-15:00.

Further, the step of obtaining the second flow rate corresponding to each second liquid respectively based on the duration of the second infusion time period and the second intake of the second liquid may include the following steps:
Obtaining the duration of the second infusion period by calculating the sum of the at least one second time period, in which one second time period defines an uninterrupted interval of time for infusing the second liquid into the target;
Obtaining the second flow rate of each second liquid by dividing the second intake and the duration of the second infusion period of the second liquid.

In this embodiment, when the second infusion time period includes two second time periods, and the two second time periods are 9:00-11:00 and 14:00-16:00, the duration of the second infusion period may be 240 min. If the second intake is 480 ml, the second flow rate may be 2 ml/min. Then the liquid infusion system may deliver the second liquid to the patient at a flow rate of 2 ml/min via the infusion pump during two time periods, 9:00-11:00 and 14:00-16:00.

### Embodiment 2

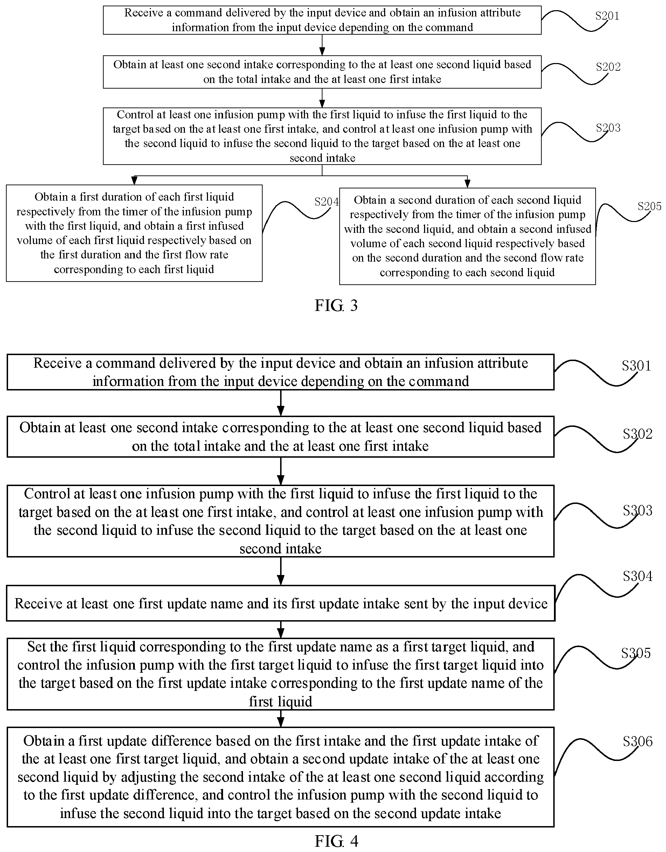

As shown in FIG 3, the invention provides a liquid infusion control method applied to a liquid infusion system. The liquid infusion system includes a control device, an input device and an infusion pump. The liquid infusion system further includes a corresponding timer for each infusion pump. The method runs in the control device and includes the following steps:
S201: when a command delivered by the input device is received, infusion attribute information may be obtained from the input device depending on the command. The command indicates that the input device has generated infusion attribute information. The infusion attribute information indicates an infusion of at least one first liquid and at least one second liquid into the target. Each first liquid is kept in one infusion pump, and each second liquid is kept in another infusion pump. The infusion attribute information includes parameter information, and the parameter information includes a total intake for a predetermined time period and at least one first intake corresponding to the at least one first liquid. The first intake defines an infusion volume of the first liquid infused into the target. The total intake defines an infusion volume of the at least one first liquid and the at least one second liquid infused into the target.

This step is consistent with S101 in Embodiment 1, so it is not repeated here.

S202: at least one second intake corresponding to the at least one second liquid may be obtained based on the total intake and the at least one first intake. The second intake defines an infusion volume of the second liquid infused into the target.

This step is consistent with S102 in Embodiment 1, so it is not repeated here.

S203: at least one infusion pump with the first liquid is controlled to infuse the first liquid into the target based on the at least one first intake, and at least one infusion pump with the second liquid infuses the second liquid into the target based on the at least one second intake.

This step is consistent with S103 in Embodiment 1, so it is not repeated here.

S204: a first duration of each first liquid may be obtained respectively from the timer of the infusion pump with the first liquid, then the first infused volume of each first liquid may be obtained respectively based on the first duration and the first flow rate corresponding to each first liquid. The first duration defines an infused time of the first liquid infused into the target. The first infused volume defines a liquid volume of the first liquid that has been infused into the target.

In this step, the timer for each infusion pump with the first liquid is configured to record the first duration of each liquid infusion. The first infused volume for each first liquid may be obtained based on the first duration and the first flow rate of each first liquid. The medical staff member is able to be informed of the current amount of each first liquid that has been infused into the patient based on the first infused volume for each first liquid.

Further, after obtaining the first infused volume of each first liquid respectively based on the first duration and the first flow rate corresponding to each first liquid, the method further includes the following steps:
Loading the ingredient information, the first intake, the first flow rate, the first duration, and the first infused volume of the at least one first liquid into a preset display module; and/or,
Loading the first infusion time period into the display module.

In this embodiment, the ingredient information, the first intake, the first flow rate, the first duration, the first infused volume and the first infusion time period of the first liquid are displayed on the display module for comprehensively showing the infusion situation of the first liquid to the medical staff member. The display module may be mounted on the liquid infusion system.

In this embodiment, the display module is a liquid crystal display module or a digital display liquid crystal module. The liquid crystal display module, abbreviated as "LCM", is a component that is an assembly of liquid crystal display devices, connectors, integrated circuits, PCB circuit boards, backlighting, and structural components together. Digital LCD module is a kind of functional component made up of segment-type liquid crystal display device and special integrated circuit, which can only display numbers and some identification symbols.

S205: a second duration of each second liquid may be obtained respectively from the timer of the infusion pump with the second liquid, then the second infused volume of each second liquid may be obtained respectively based on the second duration and the second flow rate corresponding to each second liquid. The second duration defines an infused time of the second liquid infused into the target. The second infused volume defines a liquid volume of the second liquid that has been infused into the target.

In this step, the timer for each infusion pump with the second liquid is configured to record the second duration of each liquid infusion. The second infused volume for each second liquid may be obtained based on the second duration and the second flow rate of each second liquid. The medical staff member is able to be informed of the current amount of each second liquid that has been infused into the patient based on the second infused volume for each second liquid.

Further, after obtaining the second infused volume of each second liquid respectively based on the second duration and the second flow rate corresponding to each second liquid, the method further includes the following steps:
Loading the ingredient information, the second intake, the second flow rate, the second duration, and the second infused volume of the at least one second liquid into a preset display module; and/or,
Loading the second infusion time period into the display module.

In this embodiment, the ingredient information, the second intake, the second flow rate, the second duration, the second infused volume and the second infusion time period of the second liquid are displayed on the display module for comprehensively showing the infusion situation of the second liquid to the medical staff member. The display module may be mounted on the liquid infusion system.

In this embodiment, the display module is a liquid crystal display module or a digital display liquid crystal module. The liquid crystal display module, abbreviated as "LCM", is a component that is an assembly of liquid crystal display devices, connectors, integrated circuits, PCB circuit boards, backlighting, and structural components together. Digital LCD module is a kind of functional component made up of segment-type liquid crystal display device and special integrated circuit, which can only display numbers and some identification symbols.

### Embodiment 3

As shown in FIG 4, the invention provides a liquid infusion control method applied to a liquid infusion system. The liquid infusion system includes a control device, an input device and an infusion pump. The liquid infusion system further includes a corresponding timer for each infusion pump. The method runs in the control device and includes the following steps:
S301: When a command delivered by the input device is received, infusion attribute information may be obtained from the input device depending on the command. The command indicates that the input device has generated infusion attribute information. The infusion attribute information indicates an infusion of at least one first liquid and at least one second liquid into the target. Each first liquid is kept in one infusion pump, and each second liquid is kept in another infusion pump. The infusion attribute information includes parameter information, and the parameter information includes a total intake for a predetermined time period and at least one first intake corresponding to the at least one first liquid. The first intake defines an infusion volume of the first liquid infused into the target. The total intake defines an infusion volume of the at least one first liquid and the at least one second liquid infused into the target.

This step is consistent with S101 in Embodiment 1, so it is not repeated here.

S302: at least one second intake corresponding to the at least one second liquid may be obtained based on the total intake and the at least one first intake. The second intake defines an infusion volume of the second liquid infused into the target.

This step is consistent with S102 in Embodiment 1, so it is not repeated here.

S303: at least one infusion pump with the first liquid is controlled to infuse the first liquid into the target based on the at least one first intake, and at least one infusion pump with the second liquid infuses the second liquid into the target based on the at least one second intake.

This step is consistent with S 103 in Embodiment 1, so it is not repeated here.

S304: at least one first update name and its first update intake sent by the input device are received.

In this step, the medical staff member may modify the first intake of the first liquid that is currently to be or is being infused by entering the first update name and the first update intake via the input device. The at least one first update name and its corresponding first update intake sent by the medical staff member via the input device may be received. The first update name is a name of the first liquid whose intake needs to be changed. The first update intake is a changed intake of the first liquid whose intake needs to be changed.

S305: the first liquid corresponding to the first update name is set as a first target liquid, and the infusion pump with the first target liquid may be controlled to infuse the first target liquid into the target based on the first update corresponding to the first update name of the first liquid.

In this step, a dynamic adjustment of the first liquid is realized by updating the first intake of the first liquid. It is realized that in a complex rescue situation, the medical staff member may dynamically adjust the amount of the first liquid (the main drug) to be infused according to the actual situation of the patient, and ensure that the first intake of the first liquid to be infused to the patient via the infusion pump is controllable.

In this embodiment, the step of controlling the infusion pump with the first target liquid to infuse the first target liquid into the target based on the first update intake corresponding to the first update name of the first liquid further includes the following steps:
Obtaining a first duration of each first target liquid respectively from the timer corresponding to each infusion pump with the first target liquid, and obtaining a first infused volume of each first target liquid respectively based on the first duration and the first flow rate of each first target liquid, in which the first duration defines an infused time of the first target liquid infused into the target. The first infused volume defines a liquid volume of the first target liquid that has been infused into the target;
Obtaining a first volume of each first target liquid to be infused respectively based on the first update intake and the first infused volume of each first target liquid, in which the first volume to be infused defines the remaining volume of the first target liquid infused into the target;
Obtaining a first update time period for each first target liquid respectively based on the first flow rate and the first volume to be infused, and controlling the infusion pump with the first target liquid to infuse each first target liquid with the remaining volume into the target during the first update time period according to the first flow rate.

Alternatively, a first time difference between the duration of the first infusion time period and the first duration is calculated, and a first update flow rate of the first target liquid may be obtained based on the first volume to be infused and the first time difference. The infusion pump with the first target liquid may infuse the remaining volume into the target during the first time difference according to the first update flow rate.

In this embodiment, the first volume to be infused to the patient subsequently may be obtained based on the first infused volume of the first liquid corresponding to the first update name and the first updated intake that actually needs to be infused to the patient, as subsequently adjusted by the medical staff member.

After obtaining the first volume to be infused, as one option, the first liquid may be infused to the patient in a manner in which the first flow rate remains unchanged and the duration of the first infusion time period is adjusted; alternatively, the first liquid may be infused to the patient in a manner in which the duration of the first infusion time period remains unchanged and the first flow rate is updated. The technical effect of adjusting the first liquid from both the time dimension and the flow rate dimension is realized to adapt to a variety of complex application scenarios. For example, if the flow rate of some medicines is so fast that it might cause side effects, the infusion time can be prolonged by adjusting the first infusion time period of the first liquid. Alternatively, when there is a need to increase the first intake of the first liquid and time is very short due, for example, to the subsequent need to perform other resuscitation measures on the patient, an increase in the first flow rate may be used, which may achieve the effect of increasing the first intake of the first liquid to the patient without altering the original first infusion time period.

Further, after the step of infusing the at least one first liquid and the at least one second liquid into the target according to the at least one first intake and the at least one second intake, respectively, the method further includes the following steps:
Loading the updated ingredient information, the first intake, the first duration and the first infused volume of the at least one first liquid into a preset display module, and loading the first update flow rate of the at least one first liquid into the display module, and/or,
Loading the first infusion time period into the display module.

This embodiment is configured to comprehensively display the infusion of the first liquids to a medical staff member. The display module may be mounted on the liquid infusion system. In this embodiment, the display module is a liquid crystal display module or a digital display liquid crystal module. The liquid crystal display module, abbreviated as "LCM", is a component that is made of liquid crystal display devices, connectors, integrated circuits, PCB circuit boards, backlighting, and structural components together. Digital LCD module is a kind of functional component made up of segment-type liquid crystal display device and special integrated circuit, which can only display numbers and some identification symbols.

S306: a first update difference is obtained based on the first intake and the first update intake of the at least one first target liquid, and then a second update intake of the at least one second liquid is obtained by adjusting the second intake of the at least one second liquid according to the first update difference, and then the infusion pump with the second liquid is controlled to infuse the second liquid into the target based on the second update intake.

In this step, the total intake to be infused is constant for a patient, if the total intake to be infused is too small, the purpose of the treatment is not achieved, but if the total intake to be infused is too much, the patient may suffer from a medical error due to the infusion of too much liquid.

As the intake of the first target liquids changes, the second intake of the at least one second liquid intake needs to be dynamically adjusted based on the first update difference and the total intake to ensure that the total intake remains unchanged, which in turn helps to ensure the reliability and stability of the liquid infusion.

In one embodiment, the step of obtaining the first update difference based on the first intake and the first update intake of the at least one first target liquid includes the following steps:
Obtaining an original total intake of the first liquids by calculating the sum of the first intake of the first liquids;
Obtaining an updated total intake of the first liquids by calculating the sum of the first intake of the first liquids whose intake has not changed and the first updated intake of the first liquids whose intake has changed.

Obtaining the first update difference by calculating the difference between the original total intake and the updated total intake.

In this embodiment, the first intake of each of the first liquids is summed to obtain the original total intake.

If only part of the first liquids is the first target liquid whose intake has changed, the updated total intake may be obtained by calculating the sum of the first intake of the first liquids whose intake has not changed and the first updated intake of the first target liquids whose intake has changed. If all of the first liquids are first target liquids, the sum of the first updated intake of the first target liquids is calculated to obtain the total update intake.

The first update difference is obtained by calculating the difference between the original total intake and the updated total intake, and the first update difference may be configured to define a change in the first total intake of all the first liquids so as to facilitate subsequent adjustment of the second total intake of all the second liquids in accordance with the change.

In one embodiment, the step of obtaining the second update intake of the at least one second liquid by adjusting the second intake of the at least one second liquid according to the first update difference includes the following steps:
According to a preset dynamic rule or a received adjustment request, setting a corresponding second liquid as a second target liquid;
Obtaining the second update intake of the second target liquids respectively by adjusting the intake of the second target liquid according to the first update difference.

In the embodiment, the dynamic rule is a preset adjustment rule for the second liquids. For example, if the dynamic rule defines one of the second liquids as the second target liquid, such as a glucose solution, then glucose solution may be set as the second target liquid. Two or more second liquids may also be defined in the dynamic rule as the second target liquid, such as glucose solution and saline, in which case glucose solution and saline may be set as the second target liquid.

The adjustment request states the name of the second liquid for which the intake needs to be adjusted, and the second liquid corresponding to the name of the adjustment request is set as the second target liquid. For example, if the name in the adjustment request is glucose solution, glucose solution is set as the second target liquid.

Specifically, the step of adjusting the intake of the second target liquid according to the first update difference to obtain the second update intake of the second target liquid includes the following steps:
If there is one and only one second target liquid, obtaining a second update intake for the second target liquid by subtracting the second intake from the first update difference;
If the second target liquid has two or more, obtaining a first unit difference by dividing the first update difference with the number of the second target liquid. The second update intake for each second target liquid may be obtained by subtracting the second intake of each second target liquid from the first unit difference, respectively.

Alternatively, a difference for each second target liquid may be obtained respectively based on a preset target ratio and the first updated difference. The second update intake for each second target liquid may be obtained by subtracting the second intake of each second target liquid from the difference. The target ratio defines the adjustment ratio of two or more second target liquids. For example, the first updated difference is 1 L, and the two second target liquids are glucose solution and saline, and the target ratio is: glucose solution: saline = 1:3, then the difference for glucose solution is 250 ml and the difference for saline is 750 ml.

In this embodiment, the step of controlling the infusion pump with the second liquid to infuse the second liquid into the target based on the second update intake further includes the following steps:
Obtaining a second duration of each second liquid respectively from the timer corresponding to each infusion pump with the second liquid, and obtaining a second infused volume of each second liquid based on the second duration and the second flow rate of each second liquid, in which the second duration defines an infused time of the second liquid infused into the target, and the second infused volume defines a liquid volume of the second liquid that has been infused into the target;
Obtaining a second volume to be infused of each second liquid respectively based on the second update intake and the second infused volume of each second liquid, in which the second volume to be infused defines the remaining volume of the second liquid infused to the target;
Obtaining a second update time period for each second liquid respectively based on the second flow rate and the second volume to be infused, in which the infusion pump with the second liquid may infuse the remaining volume into the target during the second update time period according to the second flow rate; or
Calculating a second time difference between the length of the second infusion time period and the second duration; obtaining a second update flow rate of the second liquid may be obtained based on the second volume to be infused and the second time difference; and controlling the infusion pump with the second liquid to infuse each second liquid with the remaining volume into the target during the second time difference according to the second update flow rate.

In this embodiment, the second volume to be infused to the patient subsequently may be obtained based on the second infused volume of the second liquid corresponding to the second update name and the second updated intake that actually needs to be infused to the patient, as subsequently adjusted by the medical staff member.

After obtaining the second volume to be infused, as one option, the second liquid can be infused to the patient in a manner in which the second flow rate remains unchanged and the length of the second infusion time period is adjusted; alternatively, the second liquid can be infused to the patient in a manner in which the length of the second infusion time period remains unchanged and the second flow rate is updated. The technical effect of adjusting the second liquid from both the time dimension and the flow rate dimension is realized to adapt to a variety of complex application scenarios. For example, if the flow rate of some medicines is so fast that it might cause side effects, the infusion time can be prolonged by adjusting the second infusion time period of the second liquid. Alternatively, when there is a need to increase the second intake of the second liquid and time is very short due, for example, to the subsequent need to perform other resuscitation measures on the patient, an increase in the second flow rate may be used, which may achieve the effect of increasing the second intake of the first liquid to the patient without altering the original second infusion time period.

Further, after the step of infusing the at least one first liquid and the at least one second liquid into the target according to the at least one first intake and the at least one second intake, respectively, the method further includes the following steps:
Loading the updated ingredient information, the second intake, the second duration and the second infused volume of the at least one second liquid into a preset display module, and loading the second update flow rate of the at least one second liquid into the display module; and/or,
Loading the second infusion time period into the display module.

This embodiment is configured to comprehensively display the infusion of the second liquids to a medical staff member. The display module may be mounted on the liquid infusion system. In this embodiment, the display module is a liquid crystal display module or a digital display liquid crystal module. The liquid crystal display module, abbreviated as "LCM", is a component that is an assembly of liquid crystal display devices, connectors, integrated circuits, PCB circuit boards, backlighting, and structural components together. A digital LCD module is a kind of functional component made up of segment-type liquid crystal display device and special integrated circuit, which can only display numbers and some identification symbols.

### Embodiment 4

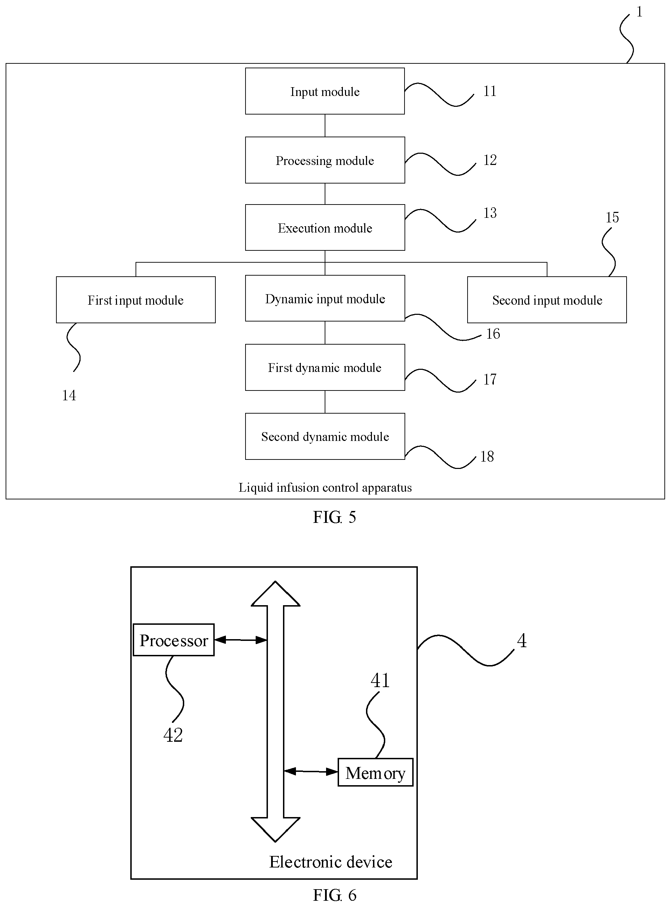

As shown in FIG 5, the invention provides a liquid infusion control apparatus applied to a liquid infusion system. The liquid infusion system includes a control device, an input device and an infusion pump. The liquid infusion control apparatus 1 installed in the control device includes the following modules.

The input module 11 is configured to receive a command delivered by the input device and obtain infusion attribute information from the input device depending on the command. The command indicates that the input device has generated infusion attribute information. The infusion attribute information indicates an infusion of at least one first liquid and at least one second liquid into the target. Each first liquid is kept in one infusion pump, and each second liquid is kept in another infusion pump. The infusion attribute information includes parameter information, and the parameter information includes a total intake for a predetermined time period and at least one first intake corresponding to the at least one first liquid. The first intake defines an infusion volume of the first liquid infused into the target. The total intake defines an infusion volume of the at least one first liquid and the at least one second liquid infused into the target.

The processing module 12 is configured to obtain at least one second intake corresponding to the at least one second liquid based on the total intake and the at least one first intake. The second intake defines an infusion volume of the second liquid infused into the target.

The execution module 13 is configured to control the at least one infusion pump with the first liquid to infuse the first liquid to the target based on the at least one first intake, and control at least one infusion pump with the second liquid to infuse the second liquid to the target based on the at least one second intake.

Optionally, the liquid infusion control apparatus 1 further includes a first input module 14 and a second input module 15.

The first input module 14 is configured to obtain a first duration of each first liquid respectively from the timer of the infusion pump with the first liquid, and then obtain the first infused volume of each first liquid respectively based on the first duration and the first flow rate of the first liquid. The first duration defines an infused time of the first liquid infused into the target. The first infused volume defines a liquid volume of the first liquid that has been infused into the target.

The second input module 15 is configured to obtain a second duration of each second liquid respectively from the timer of the infusion pump with the second liquid, and then obtain the second infused volume of each second liquid respectively based on the second duration and the second flow rate of the second liquid. The second duration defines an infused time of the second liquid infused into the target. The second infused volume defines a liquid volume of the second liquid that has been infused into the target.

Optionally, the liquid infusion control apparatus 1 may further include a dynamic input module 16, a first dynamic module 17 and a second dynamic module 18.

The dynamic input module 16 is configured to receive at least one first update name and its first update intake sent by the input device.

The first dynamic module 17 is configured to set the first liquid corresponding to the first update name as the first target liquid, and control the infusion pump with the first target liquid to infuse the first target liquid into the target based on the first update intake of the first target liquid.

The second dynamic module 18 is configured to obtain the first update difference based on the first intake and the first update intake of the at least one first target liquid, and obtain the second update intake of the at least one second liquid by adjusting the second intake of the at least one second liquid according to the first update difference, and then control the infusion pump with the second liquid to infuse the second liquid into the target based on the second update intake.

### Embodiment 5

In order to achieve the above purposes, the invention also provides an electronic device 4. The electronic device includes a processor 42 and a memory 41 communicatively connected to the processor 42. The memory stores computer-executable instructions.

The processor executes computer-executable instructions stored in memory 41 to implement the liquid infusion control method described above. The components of the liquid infusion control apparatus may be dispersed in different electronic devices, and the electronic device 4 may be a device that executes a program, such as a smartphone, a tablet, a laptop, a desktop computer, a rack-mounted server, a blade-mounted server, a tower-mounted server, or a cabinet-mounted server (including stand-alone servers, or clusters of servers made up of a plurality of application servers), and the like. The electronic device of this embodiment includes at least, but not limited to a memory 41 and a processor 42 communicatively connectable to each other via a system bus , as shown in FIG 6. It is noted that FIG 6 only illustrates an electronic device having components, but it should be understood that it is not required that all components be illustrated, and more or fewer components may alternatively be illustrated. In this embodiment, the memory 41 (a readable storage medium) includes a flash memory, a hard disk, a multimedia card, a card-type memory (SD or DX memory), a random access memory (RAM), a static random access memory (SRAM), a read-only memory (ROM), an electrically erasable programmable read-only memory (EEPROM), a programmable read-only memory (PROM), Magnetic memory, disks, CD-ROMs, and the like. In some embodiments, the memory 41 may be an internal storage unit of the electronic device, such as a hard disk or memory of the electronic device. In other embodiments, the memory 41 may also be an external storage device of the electronic device, such as a plug-in hard disk, Smart Media Card (SMC), Secure Digital (SD) card or Flash Card, which is equipped on that electronic device. Of course, the memory 41 may also include both an internal storage unit and an external storage device of the electronic device. In this embodiment, the memory 41 is typically used for storing an operating system and various types of application software installed in the electronic device, such as the program code of the liquid infusion control apparatus of Embodiment 3, and the like. In addition, the memory 41 may be used to temporarily store various types of data that have been output or will be output. The processor 42 may in some embodiments be a Central Processing Unit (CPU), controller, microcontroller, microprocessor, or other data processing chip. The processor 42 is typically used to control the overall operation of the electronic device. In this embodiment, the processor 42 is used to run program code stored in the memory 41 or to process data, such as to run a liquid infusion control apparatus to implement the liquid infusion control method of the above embodiment.

The above-described integrated modules, implemented in the form of software function modules, may be stored in a computer-readable storage medium. The above-described software function module, stored in a storage medium, includes a number of instructions to cause an electronic device (which may be a personal computer, a server or a network device) or a processor to perform some of the steps of the methods of various embodiments of the present application. It should be understood that the processor may be a central processing unit (CPU), other general purpose processor, digital signal processor (DSP), application-specific integrated circuit (ASIC), and the like. The general purpose processor may be a microprocessor or any conventional processor. The steps of the method disclosed in conjunction with the invention may be directly embodied in a hardware processor performing the completion, or performed with a combination of hardware and software modules in the processor. The memory may include a high-speed RAM memory or may also include a non-volatile storage NVM, such as at least one disk memory. The memory can also be a USB flash drive, removable hard drive, read-only memory, disk or CD-ROM.

In order to achieve the above purposes, the invention also provides a computer-readable storage medium, such as a flash memory, a hard disk, a multimedia card, a card-type memory (SD or DX memory), a random access memory (RAM), a static random access memory (SRAM), a read-only memory (ROM), an electrically erasable programmable read-only memory (EEPROM), a programmable read-only memory (PROM), magnetic memory, disks, CD-ROMs, servers, app malls, and more. The computer-readable storage medium stores computer-executable instructions, and the program realizes corresponding functions when executed by the processor 42. The computer-readable storage medium of the invention is used to store computer-executable instructions for realizing a liquid infusion control method, and realizes the liquid infusion control method of the above embodiment when executed by the processor 42.

The storage medium described above may be implemented by any type of volatile or non-volatile storage device or a combination thereof, such as static random access memory (SRAM), electrically erasable programmable read-only memory (EEPROM), erasable programmable read-only memory (EPROM), programmable read-only memory (PROM), read-only memory (ROM), magnetic memory, flash memory, disks, or CD-ROMs. The storage medium may be any available medium that can be accessed by a general-purpose or specialized computer.

For example, a storage medium is coupled to a processor such that the processor is capable of reading information from the storage medium and may write information to the storage medium. Of course, the storage medium may also be an integral part of the processor. The processor and the storage medium may be located in Application Specific Integrated Circuits (ASICs). Of course, the processor and the storage medium may also be present as discrete components in an electronic device or master device.

The invention provides a computer program product including a computer program, and the computer program being executed by a processor to implement the liquid infusion control method described above.

It should be noted that, as used herein, the terms "including", "comprising", or any other variant thereof, are intended to cover non-exclusive inclusion, such that a process, method, article, or apparatus that comprises a set of elements includes not only those elements but also other elements that are not expressly listed or that are inherent to such process, method, article or device. Without further limitation, the fact that an element is defined by the phrase "includes a ......" does not preclude the existence of another element of the same type in the process, method, article, or apparatus that includes that element.

Other embodiments of this disclosure will readily come to mind to those skilled in the art upon consideration of the specification and practice of the invention disclosed herein. This disclosure is intended to cover any variations, uses, or adaptations of the invention that follow the general principles of this invention and include means of common knowledge or customary skill in the art not disclosed herein. The specification and embodiments are to be regarded as an example only, and the true scope and spirit of this invention is indicated by the following claims.

It should be understood that the invention is not limited to the precise structure which has been described above and illustrated in the accompanying drawings, and that various modifications and changes may be made without departing from its scope. The scope of this invention is limited only by the appended claims.

## Claims

1. A liquid infusion control method applied to a liquid infusion system, wherein the liquid infusion system comprises a control device, an input device and an infusion pump; and the method runs in the control device and comprises:
receiving a command delivered by the input device and obtaining an infusion attribute information from the input device according to the command; wherein the command indicates that the input device has generated an infusion attribute information, and the infusion attribute information indicates an infusion of at least one first liquid and at least one second liquid into the target; each first liquid is kept in one infusion pump, and each second liquid is kept in another infusion pump; the infusion attribute information comprises parameter information, and the parameter information comprises a total intake for a predetermined time period and at least one first intake corresponding to the at least one first liquid; the first intake defines an infusion volume of the first liquid infused into the target, and the total intake defines an infusion volume of the at least one first liquid and the at least one second liquid infused into the target;
obtaining at least one second intake corresponding to the at least one second liquid based on the total intake and the at least one first intake; wherein the second intake defines an infusion volume of the second liquid infused into the target;
controlling at least one infusion pump with the first liquid to infuse the first liquid into the target based on the at least one first intake ; and
controlling at least one infusion pump with the second liquid to infuse the second liquid to the target based on the at least one second intake.

2. The liquid infusion control method of claim 1, wherein
the step of obtaining at least one second intake corresponding to the at least one second liquid based on the total intake and the at least one first intake comprises:
calculating the sum of the at least one first intake to obtain a total amount of the first liquid;
subtracting the total intake from the total amount of the first liquid to obtain a total amount of the second liquid;
obtaining a proportional information from the infusion attribute information and determining a second intake of each second liquid respectively; wherein the proportion information defines a proportion of each second liquid infused into the target.

3. The liquid infusion control method of claim 1, wherein
the step of controlling at least one infusion pump with the first liquid to infuse the first liquid to the target based on the at least one first intake comprises:
obtaining a first infusion time of each first liquid respectively based on a first flow rate and the first intake corresponding to each first liquid when the parameter information comprises the first flow rate;
controlling the infusion pump with the first liquid to infuse the first liquid into the target based on the first infusion time and the first flow rate; and
the step of controlling at least one infusion pump with the second liquid to infuse the second liquid to the target based on the at least one second intake, comprises:
obtaining a second infusion time of each second liquid respectively based on a second flow rate and the second intake corresponding to each second liquid when the parameter information comprises the second flow rate;
controlling the infusion pump with the second liquid to infuse the second liquid into the target based on the second infusion time and the second flow rate;
wherein the first flow rate is a speed of the first liquid infused into the target, and the second flow rate is a speed of the second liquid infused into the target.

4. The liquid infusion control method of claim 1, wherein
the step of controlling at least one infusion pump with the first liquid to infuse the first liquid to the target based on the at least one first intake comprises:
obtaining a first flow rate of each first liquid respectively based on a duration of a first infusion time period and the first intake corresponding to each first liquid when the parameter information comprises the first infusion time period;
controlling the infusion pump with the first liquid to infuse the first liquid into the target based on the first infusion time period and the first flow rate; and
the step of controlling at least one infusion pump with the second liquid to infuse the second liquid to the target based on the at least one second intake comprises:
obtaining a second flow rate of each second liquid respectively based on a duration of a second infusion time period and the second intake corresponding to each second liquid when the parameter information comprises the second infusion time period;
controlling the infusion pump with the second liquid to infuse the second liquid into the target based on the second infusion time period and the second flow rate.

5. The liquid infusion control method of claim 3 or 4, wherein the liquid infusion system comprises a timer corresponding to each infusion pump;
after the step of controlling at least one infusion pump with the first liquid to infuse the first liquid to the target based on the at least one first intake; and controlling at least one infusion pump with the second liquid to infuse the second liquid to the target based on the at least one second intake, the method further comprises:
obtaining a first duration of each first liquid respectively from the timer of the infusion pump with the first liquid; and obtaining a first infused volume of each first liquid respectively based on the first duration and the first flow rate corresponding to each first liquid; wherein the first duration defines an infused time of the first liquid infused into the target, and the first infused volume defines a liquid volume of the first liquid infused into the target; and
obtaining a second duration of each second liquid respectively from the timer of the infusion pump with the second liquid; and obtaining a second infused volume of each second liquid respectively based on the second duration and the second flow rate corresponding to each second liquid; wherein the second duration defines an infused time of the second liquid infused into the target, and the second infused volume defines a liquid volume of the second liquid infused into the target.

6. The liquid infusion control method of claim 3 or 4, wherein the liquid infusion system comprises a timer corresponding to each infusion pump;
after the step of controlling at least one infusion pump with the first liquid to infuse the first liquid to the target; and controlling at least one infusion pump with the second liquid to infuse the second liquid to the target, the method further comprises:
receiving at least one first update name and its first update intake sent by the input device;
setting the first liquid corresponding to the first update name as a first target liquid, and controlling the infusion pump with the first target liquid to infuse the first target liquid into the target based on the first update intake corresponding to the first update name of the first liquid ;
obtaining a first update difference based on the first intake and the first update intake of the at least one first target liquid, and obtaining a second update intake of the at least one second liquid by adjusting the second intake of the at least one second liquid according to the first update difference, and controlling the infusion pump with the second liquid to infuse the second liquid into the target based on the second update intake.

7. The liquid infusion control method of claim 6, wherein
the step of controlling the infusion pump with the first target liquid to infuse the first target liquid into the target based on the first update intake corresponding to the first update name of the first liquid comprises:
obtaining a first duration of each first target liquid respectively from the timer corresponding to each infusion pump with the first target liquid, and obtaining a first infused volume of each first target liquid respectively based on the first duration and the first flow rate of each first target liquid; wherein the first duration defines an infused time of the first target liquid infused into the target, and the first infused volume defines a liquid volume of the first target liquid infused into the target;
obtaining a first volume to be infused of each first target liquid respectively based on the first update intake and the first infused volume of each first target liquid; wherein the first volume to be infused defines a remaining volume of the first target liquid infused into the target;
obtaining a first update time period of each first target liquid respectively based on the first flow rate and the first volume to be infused; and controlling the infusion pump with the first target liquid to infuse each first target liquid with the remaining volume into the target during the first update time period according to the first flow rate; or
calculating a first time difference between the duration of the first infusion time period and the first duration, and obtaining a first update flow rate of the first target liquid based on the first volume to be infused and the first time difference, and controlling the infusion pump with the first target liquid infuse each first target liquid with the remaining volume into the target during the first time difference according to the first update flow rate.

8. The liquid infusion control method of claim 6, wherein
the step of obtaining the first update difference based on the first intake and the first update intake of the at least one first target liquid comprises:
calculating a sum of each first intake of the at least one first liquid to obtain an original total intake of the at least one first liquid;
calculating a sum of each non-updated first intake and each first updated intake of the at least one first liquid or calculating a sum of each first updated intake to obtain an updated total intake of the at least one first liquid;
calculating a difference between the original total intake and the updated total intake to obtain the first update difference.

9. The liquid infusion control method of claim 6, wherein
the step of obtaining the second update intake of the at least one second liquid by adjusting the second intake of the at least one second liquid according to the first update difference comprises:
setting a corresponding second liquid as a second target liquid according to a preset dynamic rule or a received adjustment request;
obtaining the second update intake of each second target liquid respectively by adjusting the intake of the second target liquid according to the first update difference.

10. The liquid infusion control method of claim 9, wherein
the step of controlling the infusion pump with the second liquid to infuse the second liquid into the target based on the second update intake, further comprises:
obtaining a second duration of each second liquid respectively from the timer corresponding to each infusion pump with the second liquid, and obtaining a second infused volume of each second liquid respectively based on the second duration and the second flow rate of each second liquid; wherein the second duration defines an infused time of the second liquid infused into the target, and the second infused volume defines a liquid volume of the second liquid infused into the target;
obtaining a second volume to be infused of each second liquid respectively based on the second update intake and the second infused volume of each second liquid; wherein the second volume to be infused defines a remaining volume of the second liquid infused to the target;
obtaining a second update time period of each second liquid respectively based on the second flow rate and the second volume to be infused; and controlling the infusion pump with the second liquid to infuse each second liquid with the remaining volume into the target during the second update time period according to the second flow rate; or
calculating a second time difference between the length of the second infusion time period and the second duration, and obtaining a second update flow rate of the second liquid based on the second volume to be infused and the second time difference, and controlling the infusion pump with the second liquid infuse each second liquid with the remaining volume into the target during the second time difference according to the second update flow rate.

11. A liquid infusion control apparatus, wherein the apparatus is applied to a liquid infusion system, the liquid infusion system comprises a control device, an input device and an infusion pump; and the apparatus is installed in the control device and comprises:
an input module configured to receive a command delivered by the input device and obtain an infusion attribute information from the input device according to the command; wherein the command indicates that the input device has generated an infusion attribute information, and the infusion attribute information is configured to indicate an infusion of at least one first liquid and at least one second liquid into the target; each first liquid is kept in one infusion pump, and each second liquid is kept in another infusion pump; the infusion attribute information comprises parameter information, and the parameter information comprises a total intake for a predetermined time period and at least one first intake corresponding to the at least one first liquid; the first intake defines an infusion volume of the first liquid infused into the target, and the total intake defines an infusion volume of the at least one first liquid and the at least one second liquid infused into the target;
a processing module configured to obtain at least one second intake corresponding to the at least one second liquid based on the total intake and the at least one first intake; wherein the second intake defines an infusion volume of the second liquid infused into the target;
an execution module configured to control at least one infusion pump with the first liquid to infuse the first liquid to the target based on the at least one first intake and control at least one infusion pump with the second liquid to infuse the second liquid to the target based on the at least one second intake.

12. An electronic device, comprising a processor and a memory communicated with the processor; wherein
the memory stores computer-executable instructions; and
the processor is configured to execute the computer-executable instructions to implement a control method in any one of claims 1 to 10.

13. A computer-readable storage medium, wherein the computer-readable storage medium stores a computer program, the computer program being executed by a processor to implement a method in any one of claims 1 to 10.
